# EUROPEAN PATENT APPLICATION

(11) **EP 4 578 310 A1**
(43) Date of publication of application: **02.07.2025**
(21) Application number: 23856220.1
(22) Date of filing: 16.06.2023
(51) Int. Cl.: A24F 40/465, A24F 40/57, H05B 6/02, H05B 6/80, A61M 15/00, A61M 11/00

(54) **AEROSOL GENERATING DEVICE AND AEROSOL GENERATING ARTICLE THEREOF, AND HEATING ASSEMBLY AND SUSCEPTOR**

(30) Priority: 26.08.2022 CN 202211037703
(71) Applicant: Shenzhen Merit Technology Co., Ltd., Shenzhen, Guangdong 518105 (CN)
(72) Inventor: HUANG, Zufu, Shenzhen, Guangdong 518105 (CN); HU, Guoqin, Shenzhen, Guangdong 518105 (CN); LI, Yu, Shenzhen, Guangdong 518105 (CN); LIANG, Feng, Shenzhen, Guangdong 518105 (CN)
(74) Representative: Westphal, Mussgnug & Partner, Patentanwälte mbB
(86) International application number: PCT/CN2023/100876
(87) International publication number: WO 2024/041128

(57) **Abstract**

The present inyention relates to an aerosol generating device and an aerosol generating article thereof, and a heating assembly and a susceptor. The susceptor comprises a single-layer sensing material, wherein the sensing material is used for generating heat based on the action of a changing magnetic field, and the sensing material is used for changing a magnetic permeability on the basis of the temperature of the susceptor. The heating assembly comprises the susceptor, and a coil which surrounds the susceptor and is used for generating a magnetic field. The aerosol generating device comprises the heating assembly. In the susceptor in the present invention, a single-layer sensing material is used, the structure of the susceptor is simple, and a magnetic permeability of the sensing material changes overa temperature, thereby facilitating the accurate temperature control of the susceptor, and improving the heating efficiency of the susceptor.

## Description

### FIELD

The invention relates to the field of atomization, in particular to an aerosol generating device, an aerosol generating article, a heating assembly, and a susceptor thereof.

### BACKGROUND

Currently, the main heating methods for heat-not-burnsmoking sets in the market are resistive heating and electromagnetic heating, among which the arrangement of heating elements in electromagnetic heating appliances is more flexible than resistive heating. No matter whether it is needle-type, chip-type, or circle, it is necessary to carry a temperature measuring wire for temperature feedback, to control the temperature variation range of the heating elements accurately, but this will reduce the application flexibility of the heating elements.

For electromagnetic heating, needle-type or chip-type or circumferential heating element is used. Traditionally, a temperature measuring film is printed on the outer surface and then connected to PCB through leads, so that the temperature of the heating element can be reflected through the feedback of the temperature measuring film. This way essentially reflects the temperature parameters of the heating element indirectly by borrowing the characteristics of other substances, which is mixed with many uncertainties, such as the heat conduction between the temperature measuring film and the heating element delays the heat exchange, and the temperature measuring film itself has heat capacity. A part of the energy is naturally consumed in the transmission process. At the same time, the process forming mode and protection mode of the temperature measuring film itself will limit the manufacturing shape of the heating element. Given these factors, the fixing of the heating element will become more complicated, which increases the structural cost and the fixing structure absorbs some energy of the heating element, reducing the efficiency of the heating element.

### SUMMARY

The technical problem to be solved by the present invention is to provide an aerosol generating device, an aerosol generating article, a heating assembly, and a susceptor thereof with a simple structure and high heating efficiency.

The technical scheme adopted by the invention to solve the technical problems is as follows: a susceptor is constructed for an aerosol generating device, and the susceptor comprises a single-layer sensing material, wherein the sensing material is used for generating heat based on the action of a changing magnetic field, and the sensing material is used for changing the magnetic permeability on the basis of the temperature of the susceptor.

Preferably, the magnetic permeability of the sensing material changes based on the change of temperature within a preset temperature range.

Preferably, the preset temperature range is greater than or equal to a first temperature value and less than or equal to a second temperature value, and the second temperature value is lower than the Curie point temperature of the sensing material.

Preferably, the magnetic permeability of the sensing material gradually increases with the gradual increase of temperature within the preset temperature range.

Preferably, the magnetic permeability of the sensing material gradually decreases with the gradual increase of temperature within the preset temperature range.

Preferably, the sensing material comprises a soft magnetic material.

Preferably, the Curie point temperature of the soft magnetic material is lower than 800 °C.

Preferably, the shape of the susceptor is blade-like, and the susceptor includes a sheet-like first body part and a first sharp end connected to the top end of the first body part.

Preferably, the susceptor is tubular.

Preferably, the susceptor is cylindrical, and the susceptor comprises a cylindrical second body part and a second sharp end connected to the top end of the second body part.

Preferably, the material of the susceptor comprises 1j85 material.

Preferably, the material of the susceptor comprises 1j77 material.

The invention also constructs a heating assembly, comprising the susceptor and a coil that surrounds the susceptor and is used for generating a magnetic field.

The invention also constructs an aerosol generating article, comprising an aerosol generating substrate and the susceptor, the susceptor is used for heating the aerosol generating substrate.

The invention also constructs an aerosol generating device, comprising a power supply assembly; and the heating assembly or the aerosol generating article; the power supply assembly is used for driving the susceptor to generate heat.

The invention has the following beneficial effects: in the susceptor in the present invention, a single-layer sensing material is used, the structure of the susceptor is simple, and a magnetic permeability of the sensing material changes over temperature, thereby facilitating the accurate temperature control of the susceptor, and improving the heating efficiency of the susceptor.

### BRIEF DESCRIPTION OF THE DRAWINGS

The invention will be further described with reference to the attached drawings and examples, in which:
Figure 1 is a schematic structural view of the first embodiment of the susceptor of the present invention;
Figure 2 is a schematic structural view of the second embodiment of the susceptor of the present invention; and
Figure 3 is a schematic structural view of the third embodiment of the susceptor of the present invention; and
Figure 4 is a schematic diagram of the change of magnetic permeability and temperature of the material of the susceptor of the present invention;

### DETAILED DESCRIPTION

To have a clearer understanding of the technical characteristics, purpose, and effect of the invention, the specific embodiments of the invention are described in detail in the attached drawings. In the following description, it should be understood that the orientation or position relationship indicated by "front", "rear", "upper", "lower", "left", "right", "longitudinal", "transverse", "vertical", "horizontal", "top", "bottom", "inside", "outside", "head", "tail", etc., is based on the orientation or position relationship shown in the attached figure, and is constructed and operated in a specific orientation. It is intended only to facilitate the description of the technical scheme, not to indicate that the device or element referred to must have a specific orientation, and therefore cannot be understood as a limitation of the invention.

It should also be noted that unless otherwise specified and limited, terms such as "installation", "connection", "link", "fixing" and "setting" should be understood broadly, for example, they can be fixed connection, detachable connection or integrated; can be a mechanical connection or an electrical connection; can be directly connected, can also be indirectly connected through an intermediary, can be the internal connection of two elements or the interaction between two elements. When an element is said to be "above" or "below" another element, the element can be "directly" or "indirectly" above the other element, or there may be one or more intervening elements. The terms "first", "second" and "third" are only for the convenience of describing the technical scheme, and cannot be understood as indicating or implying relative importance or implicitly indicating the number of indicated technical features. Therefore, features defined as "first", "second" and "third" can explicitly or implicitly include one or more such features. For those skilled in the art, the specific meanings of the above terms in the present invention can be understood according to specific situations.

In the following description, specific details, such as specific system structure and technology, are set forth for the purpose of explanation rather than limitation, to thoroughly understand the embodiments of the present invention. However, it will be apparent to one skilled in the art that the present invention may be practiced in other embodiments without these specific details. In other cases, detailed descriptions of well-known systems, devices, circuits, and methods are omitted so as not to obscure the description of the present invention with unnecessary details.

It should be noted that the crux of the problem of electromagnetic heating in the present invention mainly lies in the susceptor itself.

The basic principle of electromagnetic heating is that the changing current forms a changing magnetic field through the electromagnetic coil, and the changing magnetic field acts on the susceptor, the susceptor body generates an eddy current, and the eddy current is converted into heat. The actual electromagnetic coil body has certain attribute parameters, such as inductance, AC resistance, linear resistance, quality factor, and other parameters. When the susceptor is placed in the influence range of the electromagnetic coil, these parameters will change with the position where the susceptor is placed, size, material, volume in the influence zone and temperature of the susceptor in the influence zone except the linear resistance.

The magnetic properties of the electromagnetic heating susceptor material directly affect the heating temperature rise rate and efficiency of the susceptor. For metals with good magnetic properties, the susceptor material in the invention belongs to soft magnetic materials, and the important characteristic parameter for describing soft magnetic materials is magnetic permeability. It is found that the change of characteristic parameters of electromagnetic coils is directly related to the magnetic permeability of the susceptor.

For general susceptors, the important factor that restricts their flexible application and configuration is the need for external temperature measurement to assist, while the magnetic permeability of some soft magnetic materials changes with the temperature change of the body, so that that the temperature change being transformed into the magnetic permeability change can be established, and then the magnetic permeability change is transformed into the electrical parameter change of the coil itself, and further, the temperature change is transformed into the electrical parameter change of the coil itself. Only the change of electrical parameters of the coil needs to be measured, and the temperature value of heating can be calculated from the measured value, thus forming a complete temperature feedback link, through the link the temperature of the susceptor can be controlled. Based on this, the invention takes the temperature feedback mechanism based on soft magnetic materials as the goal, and designs a single-material soft magnetic functional material susceptor, to simplify the whole process of temperature measurement, manufacturing, and fixing of the susceptor.

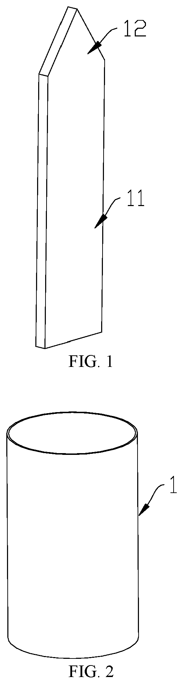

Figures 1 to 3 show a susceptor 1 for an aerosol generating device according to some embodiments of the present invention, the susceptor 1 includes a single-layer sensing material, the sensing material is used for generating heat based on the action of a changing magnetic field, and the sensing material is used for changing the magnetic permeability on the basis of the temperature of the susceptor. In some embodiments, the magnetic permeability of the sensing material changes with the temperature change within a preset temperature range, by establishing the relationship between the temperature change characteristics and the magnetic permeability change of the same material can be linear or nonlinear. The preset temperature range can be greater than or equal to a first temperature value and less than or equal to a second temperature value, and the second temperature value is lower than the Curie point temperature of the sensing material. The magnetic permeability of the temperature-sensitive layer changes with the change of temperature within the preset temperature range, and each magnetic permeability can have a corresponding temperature value, so the temperature change can be obtained according to the change of magnetic permeability.

In some embodiments, the magnetic permeability of the sensing material can gradually decrease with the gradual increase of temperature within a preset temperature range; in other embodiments, the magnetic permeability of the sensing material can gradually increase with the gradual increase of temperature within a preset temperature range.

In some embodiments, the sensing material can be made of soft magnetic material, which has a Curie temperature point, but the application range of aerosol generating substrate is within 500°C. Through the study of different soft magnetic materials, it is found that the magnetic permeability of some materials does not change obviously with temperature below the Curie temperature point, but it does change obviously with temperature only when it is close to the Curie point. However, the magnetic permeability of some materials gradually decreases with the increase of temperature below the Curie temperature point, and this change of parameter characteristics is exactly what the invention needs in practical application. Generally, the Curie point temperature of the soft magnetic material of the susceptor 1 is lower than 800°C, and it is preferable that the Curie point temperature of the sensing material used is about 400°C.

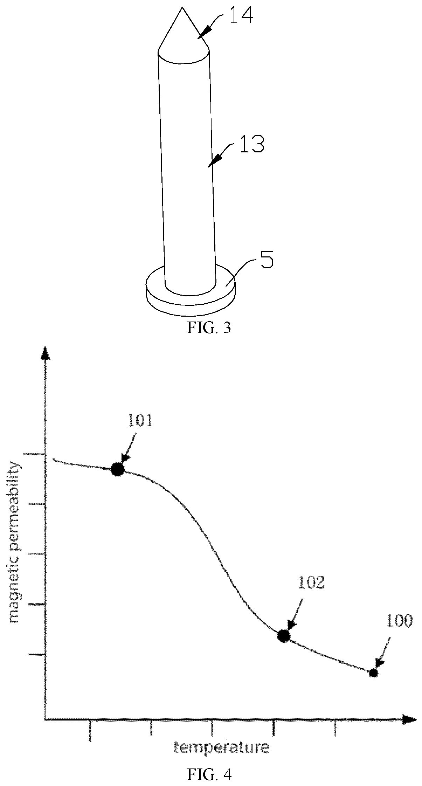

As shown in Figure 4, specifically, the soft magnetic material of the susceptor 1 has a first temperature change point 101 and a second temperature change point 102. The temperatures of the first temperature change point 101 and the second temperature change point 102 are both lower than the Curie point 100, so the corresponding temperature of the first temperature change point 101 can be selected as the first temperature value, and the corresponding temperature of the second temperature change point 102 can be selected as the second temperature value. Before the temperature of the soft magnetic material of the susceptor 1 reaches the first temperature change point 101, the magnetic permeability of the soft magnetic material of the susceptor 1 is basically unchanged. When the temperature of the soft magnetic material of the susceptor 1 reaches the first temperature change point 101 and the temperature is constantly rising, the magnetic permeability of the soft magnetic material begins to gradually and regularly decrease with the temperature increase until the temperature rises to the second temperature change point 102. After the temperature rises to the second temperature change point 102, The magnetic permeability begins to reduce the change rate, until the temperature reaches the Curie point 100 of the material, and the magnetic permeability decreases to zero or no longer changes. At this time, the material of the susceptor 1 completely loses its magnetism.

In some embodiments, the shape of the susceptor 1 can be a sheet, a tubular, a cylindrical, etc., which is not limited here.

The invention also constructs a heating assembly, including the susceptor 1 and a coil for generating a magnetic field around the susceptor 1. Further, the heating assembly further includes a fixing base 5 arranged at the lower part of the susceptor 1, and the fixing base 5 is used for fixing the susceptor 1. The invention also constructs an aerosol generating article, which includes an aerosol generating substrate and the above-mentioned susceptor, and the susceptor is used for heating the aerosol generating substrate.

The invention also constructs an aerosol generating device, including a power supply assembly and the heating assembly or the aerosol generating article, wherein the power supply assembly is used for driving the susceptor to generate heat, and the power supply assembly can include a battery. The aerosol generating device can comprise a housing, a heating assembly arranged in the housing and a battery arranged in the housing and electrically connected with two electrode leads of the heating assembly, and an aerosol generating substrate can be inserted into the housing from the top of the housing. The upper end of the heating assembly is inserted into the aerosol generating substrate, and the aerosol generating substrate is baked and heated after being electrified to raise the temperature, to form an aerosol that can be sucked by users.

Figure 1 shows a susceptor 1 according to the first embodiment of the present invention. In this embodiment, the susceptor 1 can be in the shape of a blade. The susceptor 1 includes a sheet-like first body part 11 and a first sharp end 12 connected to the top end of the first body part 11, wherein the first body part 11 has a rectangular sheet-like structure and the first sharp end 12 has a triangular sheet-like structure. The length of the bottom edge of the first sharp end 12 is the same as the length of the narrow edge of the first body part 11, and the first body part 11 and the first sharp end 12 may be integrally molded.

Further, the material of the susceptor 1 in this embodiment is 1j85 material. According to the requirements of the electromagnetic environment for the skin effect of the susceptor, the susceptor 1 is made into a sheet-like soft component using a pressed sheet of soft magnetic 1j85 material, one end of the susceptor 1 is fixed on the fixing base 5 of the aerosol generating device, and the other end is symmetrically chamfered, which is convenient for the insertion and guidance of the aerosol generating substrate. The Curie point of this material is about 400°C. The susceptor 1 is placed in the induction center of the electromagnetic coil. After inserting the aerosol generating substrate, a high-frequency current is applied to the coil. When reaching the temperature of the first temperature change point 101, the temperature of the first temperature change point 101 is 150°C, and the magnetic permeability of the susceptor 1 begins to decrease gradually and regularly, thus causing the electrical parameters of the coil to change accordingly. The current temperature value of the susceptor 1 can be inferred by capturing the electrical parameters by the circuit and performing data processing and matching. The alternating current has certain characteristics, and the temperature value of the susceptor 1 can be detected in real time according to this characteristic. Usually, when the aerosol substrate is heated, the target temperature of the susceptor 1 is about 300°C. Because this target temperature is less than the temperature of the second temperature change point 102 of the material, for example, the temperature of the second temperature change point 102 is 380°C, it can be ensured that the relationship between the temperature and the magnetic permeability of the susceptor 1 changes regularly during the heating of the susceptor 1 from the first temperature change point 101 to the target temperature 300°C, and that the temperature corresponds to the magnetic permeability one-to-one. Therefore, the magnetic permeability of the sensing material changes regularly with the temperature, so that the temperature value corresponding to each magnetic permeability in this temperature range can be achieved, and the temperature control accuracy can be achieved within 1°C.

Figure 2 shows a susceptor 1 according to a second embodiment of the present invention, and the susceptor 1 has a hollow tubular. Further, the material of the susceptor 1 in this embodiment is 1j85 material. According to the requirements of the electromagnetic environment for the skin effect of the susceptor, the susceptor 1 is made into a tubular part with a diameter of about 7mm and a length of about 12mm using a pressed sheet of soft magnetic 1j85 material, and one end of the tubular part is fixed on the guide, which is convenient for the aerosol generating substrate to be inserted and guided. The Curie point of this material is about 400°C, and the susceptor 1 is placed in the induction center of the electromagnetic coil. After inserting the aerosol generating substrate, the coil is connected with high-frequency current. When reaching the temperature of the first temperature change point 101, the temperature of the first temperature change point 101 is 150° C, and the magnetic permeability of the susceptor 1 begins to decrease gradually and regularly, thus causing the electrical parameters of the coil to change. The current temperature value of the susceptor 1 can be inferred by capturing the electrical parameters by the circuit and performing data processing and matching. The alternating current has certain characteristics, and the temperature value of the susceptor 1 can be detected in real time according to this characteristic. Usually, when the aerosol substrate is heated, the target temperature of the susceptor 1 is about 300°C. Because this target temperature is less than the temperature of the second temperature change point 102 of the material, for example, the temperature of the second temperature change point 102 is 380°C, it can be ensured that the relationship between the temperature and the magnetic permeability of the susceptor 1 changes regularly during the heating of the susceptor 1 from the first temperature change point 101 to the target temperature 300°C, and that the temperature corresponds to the magnetic permeability one-to-one. Therefore, the magnetic permeability of the sensing material changes regularly with the temperature, so that the temperature value corresponding to each magnetic permeability in this temperature range can be achieved, and the temperature control accuracy can be achieved within 1°C.

Figure 3 shows a susceptor according to a third embodiment of the present invention. The susceptor 1 may be cylindrical with a hollow interior and is generally a needle-type susceptor. The susceptor 1 includes a cylindrical second body part 13 and a second sharp end 14 connected to the top end of the second body part 13. Wherein the first body part 11 has a cylindrical structure, and the first sharp end 12 has a conical structure. The bottom area of the first sharp end 12 is the same as the top area of the first body part 11, and the first body part 11 and the first sharp end 12 can be integrally formed. Understandably, the shape of the susceptor 1 can also be other shapes such as rectangular parallelepiped, rod, etc., and there is no limitation here.

Further, the material of the susceptor 1 in this embodiment is 1j77 material. According to the design requirements of the needle-type susceptor 1, the susceptor 1 is made of the bar soft magnetic 1j77 material with higher heating efficiency, CNC, which is a needle-type part with a diameter of 2mm and a length of 12mm. One end of the needle-type susceptor 1 is fixed on the fixing base 5 of the aerosol generating device, and the other end is chamfered, which is convenient for the insertion and guidance of the aerosol generating substrate. The Curie point of this material is about 400°C, and the susceptor 1 is placed in the induction center of the electromagnetic coil. After inserting the aerosol generating substrate, the coil is connected with high-frequency current. When reaching the temperature of the first temperature change point 101, the temperature of the first temperature change point 101 is 160°C, and the magnetic permeability of the susceptor 1 begins to decrease gradually and regularly, thus causing the electrical parameters of the coil to change. The current temperature value of the susceptor 1 can be inferred by capturing the electrical parameters by the circuit and performing data processing and matching. The alternating current has certain characteristics, and the temperature value of the susceptor 1 can be detected in real time according to this characteristic. Usually, when the aerosol substrate is heated, the target temperature of the susceptor 1 is about 300°C. Because this target temperature is smaller than the temperature of the second temperature change point 102 of the material, for example, the temperature of the second temperature change point 102 is 360°C, it can be ensured that the relationship between the temperature of the susceptor 1 and the magnetic permeability changes regularly during the heating of the susceptor 1 from the first temperature change point 101 to the target temperature 300°C, and that the temperature corresponds to the magnetic permeability one-to-one. Therefore, the magnetic permeability of the sensing material changes regularly with the temperature, so that the temperature value corresponding to each magnetic permeability in this temperature range can be achieved, and the temperature control accuracy can be achieved within 1°C.

The susceptor of the invention adopts a single-layer sensing material, which has a simple structure, and the magnetic permeability of the sensing material changes with the temperature, which is beneficial to realize the accurate temperature control of the susceptor and improve the heating efficiency of the susceptor.

It can be understood that the above embodiment only expresses the preferred embodiment of the present invention, and its description is more specific and detailed, but it cannot be understood as limiting the patent scope of the present invention; it should be pointed out that for ordinary technicians in this field, the above technical features can be freely combined without departing from the concept of the present invention, and several modifications and improvements can be made, which are all within the scope of protection of the present invention; therefore, all equivalent transformations and modifications made with the scope of the claims of the present invention should belong to the scope of the claims of the present invention.

## Claims

1. A susceptor for an aerosol generating device, wherein the susceptor comprises a single-layer sensing material, wherein the sensing material is used for generating heat based on the action of a changing magnetic field, and the sensing material is used for changing a magnetic permeability on the basis of the temperature of the susceptor.

2. The susceptor according to claim 1, wherein the magnetic permeability of the sensing material changes based on the change of temperature within a preset temperature range.

3. The susceptor according to claim 2, wherein the preset temperature range is greater than or equal to a first temperature value and less than or equal to a second temperature value, and the second temperature value is lower than the Curie point temperature of the sensing material.

4. The susceptor according to claim 3, wherein the magnetic permeability of the sensing material gradually increases with the gradual increase of temperature within the preset temperature range.

5. The susceptor according to claim 3, wherein the magnetic permeability of the sensing material gradually decreases with the gradual increase of temperature within the preset temperature range.

6. The susceptor according to claim 2, wherein the sensing material comprises a soft magnetic material.

7. The susceptor according to claim 6, wherein the Curie point temperature of the soft magnetic material is lower than 800 °C.

8. The susceptor according to claim 1, wherein the susceptor is blade-like, and the susceptor includes a sheet-like first body part and a first sharp end connected to the top end of the first body part.

9. The susceptor according to claim 1, wherein the susceptor is tubular.

10. The susceptor according to claim 1, wherein the susceptor is cylindrical, and the susceptor comprises a cylindrical second body part and a second sharp end connected to the top end of the second body part.

11. The susceptor according to claim 8 or 9, wherein the material of the susceptor comprises 1j85 material.

12. The susceptor according to claim 10, wherein the material of the susceptor comprises 1j77 material.

13. A heating assembly, **characterized by** comprising the susceptor according to any one of the claims 1 to 12 and a coil that surrounds the susceptor and is used for generating a magnetic field.

14. An aerosol generating article, **characterized by** comprising an aerosol generating substrate and the susceptor according to any one of the claims 1-12, wherein the susceptor is used for heating the aerosol generating substrate.

15. An aerosol generating device, comprising a power supply assembly; and the heating assembly according to claim 13, or the aerosol generating article according to claim 14; the power supply assembly is used for driving the susceptor to generate heat.
